# EUROPEAN PATENT APPLICATION

(11) **EP 3 550 320 A1**
(43) Date of publication of application: **09.10.2019**
(21) Application number: 18165877.4
(22) Date of filing: 05.04.2018
(51) Int. Cl.: G01R 33/56, G01R 33/485, G01R 33/50, G01R 33/48, A61B 5/055, A61N 5/10

(54) **MIMICKING MAGNETIC RESONANCE IMAGING CHARACTERISTICS USING POST-PROCESSING**

(71) Applicant: Koninklijke Philips N.V., 5656 AE Eindhoven (NL)
(72) Inventor: VESANEN, Panu Tapani, 5656 AE Eindhoven (NL); WARNER, Lizette, 5656 AE Eindhoven (NL); TANTTU, Jukka Ilmari, 5656 AE Eindhoven (NL)
(74) Representative: de Haan, Poul Erik

(57) **Abstract**

The invention relates to a magnetic resonance imaging data processing system (100). The magnetic resonance imaging data processing system (100) is configured to receive first magnetic resonance imaging data (170) acquired by a first magnetic resonance imaging system (200) and having a first set of imaging characteristics. A post-processing filtering operation is applied to the first magnetic resonance imaging data (172) generating emulated magnetic resonance imaging data (176) having a set of emulated imaging characteristics. The emulated set of imaging characteristics mimics a second set of imaging characteristics (174) of second magnetic resonance imaging data (172) acquired by a second magnetic resonance imaging system (306) different from the first magnetic resonance system. (200) The emulated magnetic resonance imaging data (176) is provided for a comparison with the second magnetic resonance imaging data (172).

## Description

### FIELD OF THE INVENTION

The invention relates to the field of magnetic resonance imaging.

### BACKGROUND OF THE INVENTION

A large static magnetic field is used by Magnetic Resonance Imaging (MRI) scanners to align the nuclear spins of atoms as part of the procedure for producing images within the body of a patient. This large static magnetic field is referred to as the B0 field.

During an MRI scan, Radio Frequency (RF) pulses generated by a transmitter coil cause perturbations to the local magnetic field, and RF signals emitted by the nuclear spins are detected by a receiver coil. These RF signals are used to construct the MRI images. These coils can also be referred to as antennas. Further, the transmitter and receiver coils can also be integrated into a single transceiver coil that performs both functions. It is understood that the use of the term transceiver coil also refers to systems where separate transmitter and receiver coils are used. The transmitted RF field is referred to as the B1 field.

MRI scanners are able to construct images of either slices or volumes. A slice is a thin volume that is only one voxel thick. A voxel is a small volume over which the MRI signal is averaged, and represents the resolution of the MRI image. A voxel may also be referred to as a pixel herein.

For different applications different MRI systems with different MRI scanners are used. Different hardware features may result in differing image characteristics. For example, the filed strengths of the static magnetic fields generated by different MRI scanners may significantly differ, resulting in magnetic resonance images with different imaging characteristics. Commercial MRI systems may e.g. be available with B0 fields in the range of 0.2 T to 7 T.

### SUMMARY OF THE INVENTION

The invention provides for a magnetic resonance imaging data processing system, a computer program product, and a method in the independent claims. Embodiments are given in the dependent claims.

In one aspect, the invention relates to a magnetic resonance imaging data processing system. The magnetic resonance imaging data processing system comprises a memory storing machine executable instructions comprising instructions for a post-processing filtering operation. The magnetic resonance imaging data processing system further comprises a processor for controlling the magnetic resonance imaging data processing system. Execution of the machine executable instructions by the processor causes the processor to control the magnetic resonance imaging data processing system to receive first magnetic resonance imaging data acquired by a first magnetic resonance imaging system. The first magnetic resonance imaging data are characterized by a first set of imaging characteristics. The first set of imaging characteristics comprises one or more of the following: a first signal-to-noise-ratio, a first image contrast, a first image distortion and a first chemical shift.

Execution of the machine executable instructions further causes the processor to apply the post-processing filtering operation to the first magnetic resonance imaging data. Thereby, emulated magnetic resonance imaging data is generated. The emulated magnetic resonance imaging data is characterized by a set of emulated imaging characteristics. The set of emulated imaging characteristics comprises one or more of the following: an emulated signal-to-noise-ratio, an emulated image contrast, an emulated image distortion and an emulated chemical shift. The emulated set of imaging characteristics mimics a second set of imaging characteristics characterizing second magnetic resonance imaging data. The second magnetic resonance data is acquired by a second magnetic resonance imaging system different from the first magnetic resonance system. The second set of imaging characteristics comprises one or more of the following: a second signal-to-noise-ratio, a second image contrast, a second image distortion and a second chemical shift.

Furthermore, execution of the machine executable instructions causes the processor to provide the emulated magnetic resonance imaging data for a comparison with the second magnetic resonance imaging data. The comparison comprising displaying a first magnetic resonance image reconstructed using the emulated magnetic resonance imaging data and a second magnetic resonance image reconstructed using the second magnetic resonance imaging data.

According to embodiments, the image quality of images reconstructed using magnetic resonance imaging data acquired by the second MRI system may be matched using the first MRI system and modifying SNR, image contrast, image distortion and/or chemical shift of magnetic resonance imaging data acquired by the first MRI system by applying a post-processing operation.
For example, the image quality achieved by means of MRI systems which are optimized acquiring magnetic resonance imaging data used for reconstruction diagnostic images may often be higher than the image quality achievable by an MRI system which is used in combination with radiational systems, like a radiation delivery system. In the later case, additional constraints have to be observed, which may e.g. result in a smaller main magnetic field.

According to embodiments, the first MRI system may be an MRI system optimized for diagnostic purposes being configured for generating a main magnetic field with a larger field strength than the second MRI system. The second MRI system may e.g. be part of a MRI-guided radiation delivery system. For example, the first MRI system may be a 3 T MRI system used for preparing a dose plan, while the second MRI system may be a 1.5 T MRI system, e.g. of a MRI-guided LINAC. The second MRI system may be used for magnetic resonance imaging during execution of the prepared dose plan.

For example, in the setting of radiation therapy simulation with an MRI system and radiation delivery with a combined MRI-guided LINAC system, there may be a need for providing images of similar image quality by both systems, despite of possible differences in e.g. operating field strength.

According to embodiments, an MRI system with a first set of hardware specifications, e.g. field strength, gradient strength, etc., is configured to acquire MRI data and reconstruct MRI images having emulated image properties matching image properties of MRI images reconstructed from MRI data acquired with a second MRI system having a second set of hardware specifications different from the first set of hardware specifications.

Typically, a first MRI system used for diagnostics, treatment planning and/or radiotherapy simulation often has different hardware specifications than a second MRI system used e.g. in combination with a radiation delivery system, like a LINAC, for execution of a dose plan prepared using MRI images reconstructed using magnetic resonance imaging data acquired by the first MRI system. As a result, registration between planning MRI images and MRI images acquired for preparing the execution of the plan and/or on the fly during the execution of the plan may be challenging. According to embodiments, during the planning phase an image may be reconstructed that regarding the underlying imaging characteristics matches image reconstructed using MRI data acquired in preparation and/or on the fly during execution of the plan, which may simplify and/or improve image registration. Hereby, time may be saved during the treatment planning phase, preparation of the execution and/or during the execution of the plan.

Considering e.g. modern radiation therapy, it is divided in separated steps, comprising radiation therapy simulation and planning as well as an execution of the respective. Traditionally, the therapy may be simulated with a CT scanner.

According to embodiments, an MRI scan may be executed in addition offering a superior soft-tissue contrast relative to the CT scan. Alternatively, an MRI-only simulation may be deployed. An MRI-only planning data acquisition may have the beneficial effect that the patient is not exposed to any additional X-rays. In particular, in case such planning phases have performed repeatedly and/or in case the patient is a child, the exposition to X-rays should be minimized as far as possible.

A CT scan however uses computer-processed combinations of a plurality of X-ray measurements taken from different angles in order to produce cross-sectional, i.e. tomographic, images of an anatomical section of interest of a scanned subject.

According to embodiments, a radiation source may e.g. be provided in form of a linear accelerator (LINAC). Alternatively, may e.g. ⁶⁰Co radionuclides be used. In order to improve e.g. verification of the patient position and adjustment of the position, MRI guidance may be used for positioning and position controlling. For example, an MRI-guided LINAC system comprising a radiation delivery system with a LINAC in combination with an MRI imaging system may be used.

Consider a setting where a radiotherapy is simulated with a dedicated MRI system and the resulting dose plan executed with an MRI-guided radiation delivery system, like e.g. a LINAC system. In this setting, image quality based on the magnetic resonance imaging data provided by the two different MRI system may be inequivalent, leading to problems in e.g. image registration.

Specifically, differences in SNR and contrast may be common in case of scanners operating at different field strengths, like B0 field strength, since thermal equilibrium magnetization and relaxation times are affected by the magnetic field. For example, the B0 field strength of a diagnostic MRI system may be 3 T, while the B0 field strength generated by an MRI-guided radiation delivery system may be significantly smaller, like e.g. 1.5T or 0.35 T. More subtle effects may include image distortions due to uniformity changes due to differences in penetration of RF fields with different wavelengths, or simply because of different RF receiver coils. Also, differences in gradient coil geometry my lead e.g. to differences in the shapes and severity of fold-over artifacts or to limitations in maximum obtainable field-of-view.

In addition to the aforementioned physics-based effects above, performance differences e.g. in the gradient or RF chain may lead to differences in image quality. MRI systems comprise special software for optimizing imaging sequences in order achieve the best possible image quality for the performance and hardware behavior of that particular scanner type. Differences in gradients may be related to gradient field linearity and Eddy current related effects. Both may cause distortion, spatial and/or image intensity. The software may take into account performance of the system, like e.g. the gradient and RF performance or receiver chain characteristics, but also possible non-idealities in the system. MRI-guided radiation delivery systems may have different design requirements and constraints. Thus, the details of the sequence implementation and consequently the image appearance may differ significantly. To achieve images with matching imaging characteristics, a post processing operation may be executed to approximate the imaging characteristics of one MRI system by another MRI system.

According to embodiments, the magnetic resonance imaging data processing system may be a magnetic resonance imaging data processing system independent of the first and second magnetic resonance imaging systems. According to embodiments, the magnetic resonance imaging data processing system may comprise the first magnetic resonance imaging system and be configured to control the same. According to embodiments, the magnetic resonance imaging data processing system may comprise the second magnetic resonance imaging system and be configured to control the same.

According to embodiments, the magnetic resonance imaging data processing system further comprises a display. The execution of the machine executable instructions further causes the processor to control the magnetic resonance imaging data processing system to display the first and the second magnetic resonance images on the display. The first and the second magnetic resonance images may e.g. be displayed to control and adjust setting of the second MRI system and/or a radiation source for delivering radiation or another medical instrument. In particular, the first MRI image may be used as a reference, e.g. for positioning a subject. Using the first and second MRI image, it may e.g. be controlled that a current position of the subject coincides with a predefined position. In case of deviations, the same may be determined by the comparison of the first and second images and adjustments of the position may be initiated and/or executed. The second MRI image may e.g. be reconstructed from second magnetic resonance data acquired during preparation of the radiation delivery and/or during the delivering itself.

According to embodiments, the first magnetic resonance imaging data are received in form of a third magnetic resonance image, i.e. in form of a non-filtered magnetic resonance image from the first magnetic resonance imaging system, and the emulated magnetic resonance imaging data are generated in form of the first magnetic resonance image. Thus, the post-processing filtering operation may be applied to MRI images rather than MRI raw data.

According to embodiments, the generating of the emulated magnetic resonance imaging data with the emulated signal-to-noise-ration comprises adding noise to the first magnetic resonance imaging data in order to reduce the first signal-to-noise-ration and to match the second signal-to-noise-ration. Thus, the high signal-to-noise-ration of first MRI data acquired by the first MRI system may be reduced to match a lower signal-to-noise-ration of the second MRI system.

According to embodiments, the SNR may be artificially reduced e.g. by adding Gaussian noise to the image data in the reconstruction. According to further embodiments, the SNR may be improved using signal averaging, i.e. by averaging over a plurality of magnetic resonance imaging data acquired for identical sampling points.

According to embodiments, the generating of the emulated magnetic resonance imaging data with the emulated image contrast comprises using a combination of a T1 map and a T2 map acquired by the first magnetic resonance imaging system in order to adjust the first image contrast and to match the second image contrast. The T1 and T2 map may be comprised by the first magnetic resonance data. The combination of T1 and T2 may e.g. be a linear of a non-linear combination.

In MRI, each tissue type returns to its equilibrium state after excitation by the independent processes of Tl, i.e. spin-lattice, and T2, i.e. spin-spin, relaxation. In order to generate a T1-weighted image, i.e. an image highlighting tissue types for which T1 relaxation dominates, magnetization is allowed to recover before measuring the MRI signal by suitably controlling the repetition time (TR). In order to generate a T2-weighted image, magnetization is allowed to decay before measuring the MRI signal by suitably controlling the echo time (TE). Thus, by controlling repetition and echo time used for the data acquisition, the contrast of the resulting image can be controlled. By suitably controlling TR and TE it maybe possible to reach a desired contrast between any two tissue types with different relaxation behavior.

When the magnetic resonance data acquired by the first magnetic resonance system comprise a T1 map as well as a T2 map, it may be possible to reach a desired contrast between any two tissue types with different relaxation behavior using the T1 map and the T2 map.

According to embodiments, the generating of the emulated magnetic resonance imaging data with the emulated chemical shift comprises applying one of the following to adjust the first chemical shift and to match the second chemical shift: a fat suppression and a water suppression.

For example, the first magnetic resonance imaging data may comprise a separate fat MRI data and water MRI data using a Dixon approach, e.g. a single point or multi-point Dixon approach. Using a combination of those separate fat MRI data and water MRI data, e.g. in form of a separate fat image and water image, any chemical shift signal-to-noise-ration may be emulated by a combination of the respective two separate datasets.

Above, it has been described how to reach a desired contrast between a chosen pair of tissue types with different relaxation behavior using the T1 map and the T2 map. If neither of these two tissue types is fat, it may additionally be possible to independently reduce the fat induced signal by an arbitrary amount, e.g. by a FatSat pulse with a suitable flip angle less than 90° or by a modified Dixon reconstruction aiming for only partial fat suppression. On the other hand, the fat induced signal may be increased compared to other tissue types by applying a water suppression using e.g. the same aforementioned techniques. Thus, by combining a linear combination of the T1 map and the T2 map with an independent adjustment of the fat induced signal, it may be possible to emulate the contrast of any three tissue types, wherein a single one of these is fat, while the remaining two tissue types display a different relaxation behavior.

Image distortion, i.e. distortion of the geometry and/or intensity, of an MRI image maybe caused by a lack of homogeneity of the magnetic fields used for acquiring the magnetic resonance data. A source of geometric distortion are e.g. gradient filed non-idealities. Homogeneity refers to the uniformity of a magnetic field in the center of a scanner when no patient is present. Magnetic field homogeneity may be measured in parts per million (ppm) over a certain diameter of spherical volume (DSV). Inhomogeneity refers to a degree of lack of homogeneity of the respective magnetic fields, for example a fractional deviation of a local value of the respective magnetic field from an average value of the respective field.

A general goal, when designing and manufacturing an MRI system, is achieve a magnetic field as homogeneous as possible, especially at the core of the scanner. However, even with an ideal magnet, some intrinsic inhomogeneities may always remain, while additional inhomogeneities may be caused by the susceptibility of a subject which is positioned in the magnetic field and from which the magnetic resonance imaging data is acquired. The geometrical distortion refers to a displacement of pixel locations. Intensity distortion refers to an undesired change in the intensity or brightness of pixels/voxels, which may cause problems in determining different tissues and reduce the maximum achievable image resolution.

Such inhomogeneity effects may be compensated by post-processing using a B0 map. According to embodiments, the first magnetic resonance data comprise a B0 map acquired by the first magnetic resonance imaging system. The B0 map is used to adjust the first image distortion and to match the second image distortion. The B0 map may e.g. be acquired by a low-resolution coarse calibration reference scan.

Spatial inhomogeneity of the B 1 field, i.e. the magnetic field produced by the radio frequency coils, may e.g. case flip-angle deviations depending on the spatial position which may result in a reduced signal in these areas or an altered contrast. Such B1 inhomogeneity may e.g. arise, because the RF power is absorbed differently across the subject, due to the changing permittivity and conductivity of tissues, i.e. dielectric effects, and standing waves in tissues.

Using for example a plurality of coils, e.g. in form a phased-array coil in conjunction with parallel imaging, placed in the near vicinity or on the subject, may enable a higher SNR, but may simultaneously result in a non-uniformity of signal. The depth of penetration of coils is inversely proportional to their diameters. For small coils, this may lead to an accentuation of signals arising superficially in the subject, while signals arising deep in the subject are attenuated.

For multichannel parallel imaging it may be possible to make corrections for non-uniform receiver coil profiles using coil sensitivity maps acquired by a coil sensitivity calibration pre-scan. There are different methods known for performing such corrections based on a pre-scan, like e.g. PURE ("Phased array Uniformity Enhancement"), Prescan Normalize, CLEAR ("Constant LEvel AppeaRance") or NATURAL ("NATural Uniformity Realization Algorithm").

Distortions due to non-uniformity of the B1 magnetic field caused by receiver coil geometry, i.e. non-uniformity due to receiving, may e.g. be reduced using CLEAR reconstruction technique. Furthermore, distortions due to non-uniformity of the transmission coil geometry may be reduced as well using CLEAR. The image uniformity because of transmission may be found out by B1 mapping. If B1 mapping is performed for both scanners, and the first and second magnetic resonance data comprise the respective B 1 maps, the CLEAR reconstructed images may be modified in reconstruction with this information either to remove the non-uniformity due to transmission for both MRI systems or to set the uniformity of one MRI system to correspond to the other. In particular, the image distortion of the first magnetic resonance data, e.g. using CLEAR, may be adjusted to generate an emulated image distortion matching the image distortion of the second magnetic resonance imaging data.

According to embodiments, the first magnetic resonance data comprise a B1 map acquired by the first magnetic resonance imaging system. The B1 map is used to adjust the first image distortion and to match the second image distortion. The B1 map may e.g. be acquired by a low-resolution coarse calibration reference scan.

According to embodiments, the comparison further comprising performing an image registration of the first and the second magnetic resonance image. Based on the registration a different position and/or orientation of an anatomical structure of according to the second magnetic resonance data relative to a reference position and/or orientation defined using the first magnetic resonance data may be determined. For example, it may be determined whether and how a current position and/or orientation deviates from a position and/or orientation which forms the basis of a dose plan to be executed by a radiation delivery system.

According to embodiments, the first and the second magnetic resonance image depict an anatomical structure of interest. The first magnetic resonance image defines a spatial reference position of the anatomical structure of interest. The second magnetic resonance image provides a current spatial position of the anatomical structure of interest. The comparison further comprises determining repositioning parameters in order to adjust the current position of the anatomical structure of interest and to match the spatial reference position.
Aligning a patient for execution of a dose plan may be the more facilitated, the more magnetic resonance images used for preparing the dose plan, i.e. for deveining target areas and organs of risk, matches the magnetic resonance images acquired by the radiation delivery system.

According to embodiments, the comparison further comprises determining adjustment parameters for adjusting a dose plan for applying a radiation dose to a target comprised by the anatomical structure of interest. The adjustment parameters are configured to compensate for differences between the spatial reference position of the anatomical structure for which the dose plan is defined and the current position of the anatomical structure of interest.

Embodiments relate to MRI systems used in medical departments, i.e. diagnostic departments as well as radiotherapy departments.

According to embodiments, the magnetic resonance imaging data processing system further comprises the first magnetic resonance imaging system. The first magnetic resonance imaging system comprises a first main magnet for generating a first main magnetic field within a first imaging zone, a first magnetic field gradient system for generating a first spatially dependent gradient magnetic field within the first imaging zone, and a first radio-frequency antenna system configured for acquiring the first magnetic resonance imaging data from the first imaging zone.

The memory further stores first pulse sequence commands. The first pulse sequence commands are configured for controlling the first magnetic resonance imaging system to acquire the first magnetic resonance imaging data from the first imaging zone. The receiving of the first magnetic resonance imaging data comprises acquiring the first magnetic resonance imaging data from the first imaging zone via the radio frequency antenna system using the first pulse sequence commands.

According to embodiments, the first magnetic resonance imaging system may in addition be operable in two operation modes. In a first operation mode the first magnetic resonance imaging system may be controlled to acquire magnetic resonance imaging data with an optimized imaging quality achievable by the first magnetic resonance imaging system. In the second mode, also referred to as emulation mode, the first magnetic resonance imaging system may be controlled such that the differences of the first set of imaging characteristics relative to the second imaging characteristics are minimized. For example, a white noise RF source comprised by the first magnetic resonance imaging system may be controlled to reduce the SNR of the first magnetic resonance data to match the SNR according to the second imaging characteristics. For example, repetition and echo time for acquiring the first magnetic resonance data may be controlled in order to match the image contrast defined by the second imaging characteristics. For example, the performance, e.g. of the main magnet, the gradient coils and/or the RF coils, the first magnetic resonance imaging system may be limited in the emulation mode in order to match the lower performance of the second magnetic resonance imaging system.

Furthermore, the signal bandwidth for acquiring the first magnetic resonance data maybe controlled in order match the image distortion and chemical shift of the second magnetic resonance data.

According to embodiments, the magnetic resonance imaging data processing system further comprises the second magnetic resonance imaging system, the second magnetic resonance imaging system comprising: a second main magnet for generating a second main magnetic field within a second imaging zone, a second magnetic field gradient system for generating a second spatially dependent gradient magnetic field within the second imaging zone, and a second radio-frequency antenna system configured for acquiring the second magnetic resonance imaging data from the second imaging zone.

The memory further stores second pulse sequence commands. The second pulse sequence commands are configured for controlling the second magnetic resonance imaging system to acquire the second magnetic resonance imaging data from the second imaging zone.

Execution of the machine executable instructions further causes the processor to control the second magnetic resonance imaging system to acquire the second magnetic resonance imaging data from the second imaging zone via the second radio-frequency antenna system using the second pulse sequence commands. The second magnetic resonance image is reconstructed using the acquired magnetic resonance data.

According to embodiments, the second magnetic resonance imaging system further comprises a radiation source configured for applying one of the following to a target located within the imaging zone: X-rays and gamma rays. The radiation source may e.g. be provided by a LINAC or ⁶⁰Co radionuclides.

According to embodiments, a first magnetic field strength of the first main magnetic field generated by the first main magnet of the first magnetic resonance imaging system is larger than a second magnetic field strength of the second main magnetic field generated by the second main magnet of the second magnetic resonance imaging system. The first magnetic field strength may e.g. be a 3 T, while the magnetic field strength may e.g. be 1.5 T or even as small as 0.35 T.

In another aspect, the invention relates to a method for controlling a magnetic resonance imaging data processing system, the magnetic resonance imaging data processing system comprises a memory. The memory stores machine executable instructions comprising instructions for a post-processing filtering operation. The magnetic resonance imaging data processing system further comprises a processor for controlling the magnetic resonance imaging data processing system. Execution of the machine executable instructions by the processor causes the processor to control the magnetic resonance imaging data processing system to execute the method. The method comprises receiving first magnetic resonance imaging data acquired by a first magnetic resonance imaging system. The first magnetic resonance imaging data are characterized by a first set of imaging characteristics. The first set of imaging characteristics comprises one or more of the following: a first signal-to-noise-ratio, a first image contrast, a first image distortion and a first chemical shift.

The method further comprises applying the post-processing filtering operation to the first magnetic resonance imaging data. Thereby, emulated magnetic resonance imaging data is generated. The emulated magnetic resonance imaging data is characterized by a set of emulated imaging characteristics. The set of emulated imaging characteristics comprises one or more of the following: an emulated signal-to-noise-ratio, an emulated image contrast, an emulated image distortion and an emulated chemical shift. The emulated set of imaging characteristics mimics a second set of imaging characteristics characterizing second magnetic resonance imaging data. The second magnetic resonance data is acquired by a second magnetic resonance imaging system different from the first magnetic resonance system. The second set of imaging characteristics comprises one or more of the following: a second signal-to-noise-ratio, a second image contrast, a second image distortion and a second chemical shift.

Furthermore, the method comprises providing the emulated magnetic resonance imaging data for a comparison with the second magnetic resonance imaging data. The comparison comprising displaying a first magnetic resonance image reconstructed using the emulated magnetic resonance imaging data and a second magnetic resonance image reconstructed using the second magnetic resonance imaging data.

In another aspect, the invention relates to a computer program product comprising machine executable instructions for execution by a processor controlling a magnetic resonance imaging data processing system. The machine executable instructions comprise instructions for a post-processing filtering operation. Execution of the of the machine executable instructions by the processor causes the processor to control the magnetic resonance imaging data processing system to receive first magnetic resonance imaging data acquired by a first magnetic resonance imaging system. The first magnetic resonance imaging data are characterized by a first set of imaging characteristics. The first set of imaging characteristics comprises one or more of the following: a first signal-to-noise-ratio, a first image contrast, a first image distortion and a first chemical shift.

Execution of the machine executable instructions further causes the processor to apply the post-processing filtering operation to the first magnetic resonance imaging data. Thereby, emulated magnetic resonance imaging data is generated. The emulated magnetic resonance imaging data is characterized by a set of emulated imaging characteristics. The set of emulated imaging characteristics comprises one or more of the following: an emulated signal-to-noise-ratio, an emulated image contrast, an emulated image distortion and an emulated chemical shift. The emulated set of imaging characteristics mimics a second set of imaging characteristics characterizing second magnetic resonance imaging data. The second magnetic resonance data is acquired by a second magnetic resonance imaging system different from the first magnetic resonance system. The second set of imaging characteristics comprises one or more of the following: a second signal-to-noise-ratio, a second image contrast, a second image distortion and a second chemical shift.

Furthermore, execution of the machine executable instructions causes the processor to provide the emulated magnetic resonance imaging data for a comparison with the second magnetic resonance imaging data. The comparison comprising displaying a first magnetic resonance image reconstructed using the emulated magnetic resonance imaging data and a second magnetic resonance image reconstructed using the second magnetic resonance imaging data.

The aforementioned embodiments of the invention may enable to match magnetic resonance data acquired by a first magnetic resonance imaging system with imaging characteristics of magnetic resonance data acquired by a second magnetic resonance imaging system. The first and second magnetic resonance imaging system are different from each other and comprise different hardware specification. In particular, the B0 field generated by the first magnetic resonance imaging system may be larger than the B0 field of the second magnetic resonance imaging system.

It is understood that one or more of the aforementioned embodiments of the invention may be combined as long as the combined embodiments are not mutually exclusive.

As will be appreciated by one skilled in the art, aspects of the present invention may be embodied as an apparatus, method or computer program product. Accordingly, aspects of the present invention may take the form of an entirely hardware embodiment, an entirely software embodiment (including firmware, resident software, micro-code, etc.) or an embodiment combining software and hardware aspects that may all generally be referred to herein as a "circuit," "module" or "system." Furthermore, aspects of the present invention may take the form of a computer program product embodied in one or more computer readable medium(s) having computer executable code embodied thereon.

Any combination of one or more computer readable medium(s) may be utilized. The computer readable medium may be a computer readable signal medium or a computer readable storage medium. A 'computer-readable storage medium' as used herein encompasses any tangible storage medium which may store instructions which are executable by a processor of a computing device. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the processor of the computing device. Examples of computer-readable storage media include, but are not limited to: a floppy disk, a magnetic hard disk drive, a solid state hard disk, flash memory, a USB thumb drive, Random Access Memory (RAM), Read Only Memory (ROM), an optical disk, a magneto-optical disk, and the register file of the processor. Examples of optical disks include Compact Disks (CD) and Digital Versatile Disks (DVD), for example CD-ROM, CD-RW, CD-R, DVD-ROM, DVD-RW, or DVD-R disks. The term computer readable-storage medium also refers to various types of recording media capable of being accessed by the computer device via a network or communication link. For example, a data may be retrieved over a modem, over the internet, or over a local area network. Computer executable code embodied on a computer readable medium may be transmitted using any appropriate medium, including but not limited to wireless, wire line, optical fiber cable, RF, etc., or any suitable combination of the foregoing.

A computer readable signal medium may include a propagated data signal with computer executable code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

'Computer memory' or 'memory' is an example of a computer-readable storage medium. Computer memory is any memory which is directly accessible to a processor. 'Computer storage' or 'storage' is a further example of a computer-readable storage medium. Computer storage is any non-volatile computer-readable storage medium. In some embodiments computer storage may also be computer memory or vice versa.

A 'processor' as used herein encompasses an electronic component which is able to execute a program or machine executable instruction or computer executable code. References to the computing device comprising "a processor" should be interpreted as possibly containing more than one processor or processing core. The processor may for instance be a multi-core processor. A processor may also refer to a collection of processors within a single computer system or distributed amongst multiple computer systems. The term computing device should also be interpreted to possibly refer to a collection or network of computing devices each comprising a processor or processors. The computer executable code may be executed by multiple processors that may be within the same computing device or which may even be distributed across multiple computing devices.

Computer executable code may comprise machine executable instructions or a program which causes a processor to perform an aspect of the present invention. Computer executable code for carrying out operations for aspects of the present invention may be written in any combination of one or more programming languages, including an object-oriented programming language such as Java, Smalltalk, C++ or the like and conventional procedural programming languages, such as the "C" programming language or similar programming languages and compiled into machine executable instructions. In some instances, the computer executable code maybe in the form of a high-level language or in a pre-compiled form and be used in conjunction with an interpreter which generates the machine executable instructions on the fly.

The computer executable code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Aspects of the present invention are described with reference to flowchart illustrations and/or block diagrams of methods, apparatus (systems) and computer program products according to embodiments of the invention. It is understood that each block or a portion of the blocks of the flowchart, illustrations, and/or block diagrams, can be implemented by computer program instructions in form of computer executable code when applicable. It is further under stood that, when not mutually exclusive, combinations of blocks in different flowcharts, illustrations, and/or block diagrams may be combined. These computer program instructions may be provided to a processor of a general-purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks.

These computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks. A 'user interface' as used herein is an interface which allows a user or operator to interact with a computer or computer system. A 'user interface' may also be referred to as a 'human interface device.' A user interface may provide information or data to the operator and/or receive information or data from the operator. A user interface may enable input from an operator to be received by the computer and may provide output to the user from the computer. In other words, the user interface may allow an operator to control or manipulate a computer and the interface may allow the computer indicate the effects of the operator's control or manipulation. The display of data or information on a display or a graphical user interface is an example of providing information to an operator. The receiving of data through a keyboard, mouse, trackball, touchpad, pointing stick, graphics tablet, joystick, gamepad, webcam, headset, pedals, wired glove, remote control, and accelerometer are all examples of user interface components which enable the receiving of information or data from an operator. A 'hardware interface' as used herein encompasses an interface which enables the processor of a computer system to interact with and/or control an external computing device and/or apparatus. A hardware interface may allow a processor to send control signals or instructions to an external computing device and/or apparatus. A hardware interface may also enable a processor to exchange data with an external computing device and/or apparatus. Examples of a hardware interface include, but are not limited to: a universal serial bus, IEEE 1394 port, parallel port, IEEE 1284 port, serial port, RS-232 port, IEEE-488 port, Bluetooth connection, Wireless local area network connection, TCP/IP connection, Ethernet connection, control voltage interface, MIDI interface, analog input interface, and digital input interface.

A 'display' or 'display device' as used herein encompasses an output device or a user interface adapted for displaying images or data. A display may output visual, audio, and or tactile data. Examples of a display include, but are not limited to: a computer monitor, a television screen, a touch screen, tactile electronic display, Braille screen, Cathode ray tube (CRT), Storage tube, Bi-stable display, Electronic paper, Vector display, Flat panel display, Vacuum fluorescent display (VF), Light-emitting diode (LED) displays, Electroluminescent display (ELD), Plasma display panels (PDP), Liquid crystal display (LCD), Organic light-emitting diode displays (OLED), a projector, and Head-mounted display.

Magnetic Resonance (MR) data is defined herein as being the recorded measurements of radio frequency signals emitted by atomic spins using the antenna of a Magnetic resonance apparatus during a magnetic resonance imaging scan. A Magnetic Resonance Imaging (MRI) image or MR image is defined herein as being the reconstructed two or three-dimensional visualization of anatomic data contained within the magnetic resonance imaging data. This visualization can be performed using a computer.

### BRIEF DESCRIPTION OF THE DRAWINGS

In the following preferred embodiments of the invention will be described, by way of example only, and with reference to the drawings in which:
Fig. 1 shows an example of a magnetic resonance imaging processing system;
Fig. 2 shows an example of a computer of the magnetic resonance imaging processing system of Fig. 3;
Fig. 3 shows an example of a magnetic resonance imaging processing system;
Fig. 4 shows an example of a computer of the magnetic resonance imaging processing system of Fig. 5;
Fig. 5 shows an example of a magnetic resonance imaging processing system;
Fig. 6 illustrates an exemplary method of controlling the magnetic resonance imaging processing system of Fig. 1;
Fig. 7 illustrates an exemplary method of controlling the magnetic resonance imaging processing system of Fig. 3; and
Fig. 8 illustrates an exemplary method of controlling the magnetic resonance imaging processing system of Fig. 5.

### DETAILED DESCRIPTION OF THE EMBODIMENTS

Like numbered elements in these figures are either equivalent elements or perform the same function. Elements which have been discussed previously will not necessarily be discussed in later figures if the function is equivalent.

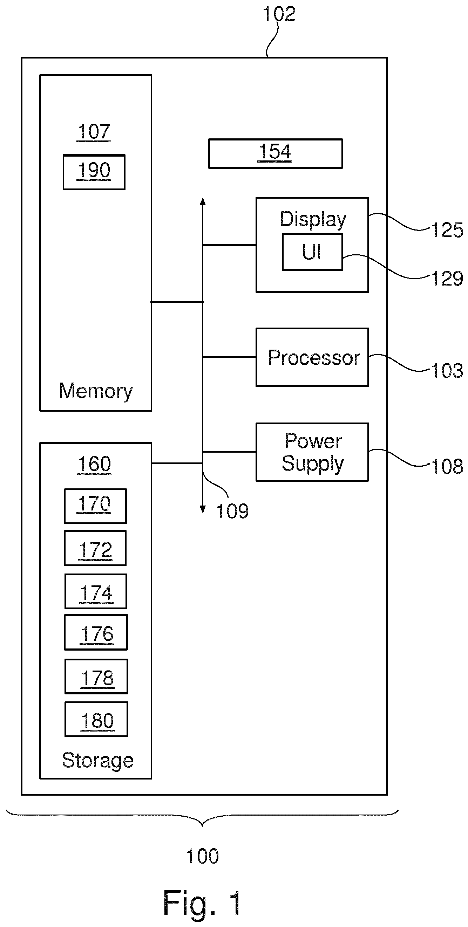

Fig. 1 shows an example of a magnetic resonance imaging data processing system 100. The magnetic resonance imaging data processing system 100 is shown to comprise a computer 102. The computer 102 comprises a processor 103, a memory 107 each capable of communicating with one or more components of the system 100. For example, components of the magnetic resonance imaging data processing system 100 are coupled to a bidirectional system bus 109.

It will be appreciated that the methods described herein are at least partly non-interactive, and automated by way of computerized systems. For example, these methods can further be implemented in software, (including firmware), hardware, or a combination thereof. In exemplary embodiments, the methods described herein are implemented in software, as an executable program. The computer 102 maybe a special or general-purpose digital computer, such as a personal computer, workstation, minicomputer, or mainframe computer.

The processor 103 is a hardware device for executing software, particularly that stored in memory 107. The processor 103 may be any custom made or commercially available processor, a central processing unit (CPU), an auxiliary processor among several processors associated with the magnetic resonance imaging data processing system 100, a semiconductor-based microprocessor (in the form of a microchip or chip set), a macroprocessor, or generally any device for executing software instructions. The processor 103 may control the operation of the magnetic resonance imaging data processing system 100. Furthermore, the processor 103 may control additional systems comprised by the magnetic resonance imaging data processing system 100. For example, the processor 103 may control a magnetic resonance imaging system and/or a magnetic resonance imaging-guided radiation delivery system to which the processor 103 is operatively connected e.g. via a hardware interface 154.

The memory 107 may include any one or combination of volatile memory elements (e.g., random access memory (RAM, such as DRAM, SRAM, SDRAM, etc.)) and nonvolatile memory elements (e.g., ROM, erasable programmable read only memory (EPROM), electronically erasable programmable read only memory (EEPROM), programmable read only memory (PROM). Note that the memory 107 may have a distributed architecture, where various components are situated remote from one another, but are accessible by the processor 103. Memory 107 may store machine readable instructions.

The magnetic resonance imaging data processing system 100 may further comprise a display device 125 which displays characters and images and the like e.g. on a user interface 129. The display device 125 may e.g. be a touch screen display device.

The magnetic resonance imaging data processing system 100 may further comprise a power supply 108 for powering the magnetic resonance imaging data processing system 100. The power supply 108 may for example be a battery or an external source of power, such as electricity supplied by a standard AC outlet.

According to embodiments, the magnetic resonance imaging data processing system 100 may be configured to receive and/or be connected to a computer readable-storage medium providing magnetic resonance imaging data acquired by one or more MRI systems. According to embodiments, the magnetic resonance imaging data processing system 100 may be configured to connect to a database and/or a network via which it may receive magnetic resonance imaging data acquired by one or more MRI systems. According to embodiments, the magnetic resonance imaging data processing system 100 may be configured to connect to one or more MRI systems via which it may receive magnetic resonance imaging data.

The connection between hardware interface 154 and an MRI system, an MRI-guided radiation delivery system or a database system may for example comprise a BUS Ethernet connection, WAN connection, Internet connection etc. The computer 102 shown in Fig. 1 and the database system may or may not be an integral part. In other terms, the computer 102 shown in Fig. 1 may or may not be external to the database system. The database system may comprise components that are controlled by the processor 103.

The processor 103 may be adapted to receive magnetic resonance imaging data from the MRI system, MRI-guided radiation delivery system or the archive system in a compatible digital form so that such magnetic resonance imaging data maybe processed and magnetic resonance images reconstructed from the received MRI data may be displayed on the display device 125. Furthermore, additional information may be received from the MRI system or MRI-guided radiation delivery system in a compatible digital form so that it may be displayed on the display device 125. Such additional information may include operating parameters, alarm notifications, and other information related to the use, operation and function of the MRI system or MRI-guided radiation delivery system.

Storage device 160 is shown as containing a first set of magnetic resonance imaging data 170 that have been acquired by a first magnetic resonance imaging system. The first set of magnetic resonance imaging data 170 is characterized by a first set of imaging characteristics. The first set of imaging characteristics comprises one or more of the following: a first signal-to-noise-ratio, a first image contrast, a first image distortion and a first chemical shift. The storage device 160 is shown as further containing a second set of magnetic resonance imaging data 172 acquired by a second magnetic resonance imaging system different from the first magnetic resonance system. The second set of magnetic resonance imaging data 172 is characterized by a second set of imaging characteristics. The second set of imaging characteristics comprises one or more of the following: a second signal-to-noise-ratio, a second image contrast, a second image distortion and a second chemical shift. The storage device 160 is shown as further containing a set of definitions 174 defining the second imaging characteristics characterizing the second set of magnetic resonance imaging data 172. The set of definitions 174 may result from an analysis of the second set of magnetic resonance imaging data 172 by the magnetic resonance imaging data processing system 100 or the set of definitions 174 may have been received by magnetic resonance imaging data processing system 100 together with the second set of magnetic resonance imaging data 172 or independently of the same. The storage device 160 is shown as further containing a set of emulated magnetic resonance imaging data 176. The set of emulated magnetic resonance imaging data 176 results from applying a post-processing filtering operation to the first magnetic resonance imaging data 170. The set of emulated magnetic resonance imaging data 176 is characterized by a set of emulated imaging characteristics. The set of emulated imaging characteristics comprises one or more of the following: an emulated signal-to-noise-ratio, an emulated image contrast, an emulated image distortion and an emulated chemical shift. The emulated set of imaging characteristics mimicking a second set of imaging characteristics as defined by the set of definitions 174. The storage device 160 is shown as further containing a first magnetic resonance image 178 reconstructed using the set of emulated magnetic resonance imaging data 176 as well as a second magnetic resonance image 180 reconstructed using the second set of magnetic resonance imaging data 172.

The memory 107 is shown as containing a post-processing filtering module 190. The post-processing filtering module 190 contains machine executable instructions an execution of which by the processor may cause the processor 103 to execute a post-processing filtering operation, e.g. on the first set of magnetic resonance imaging data 170.

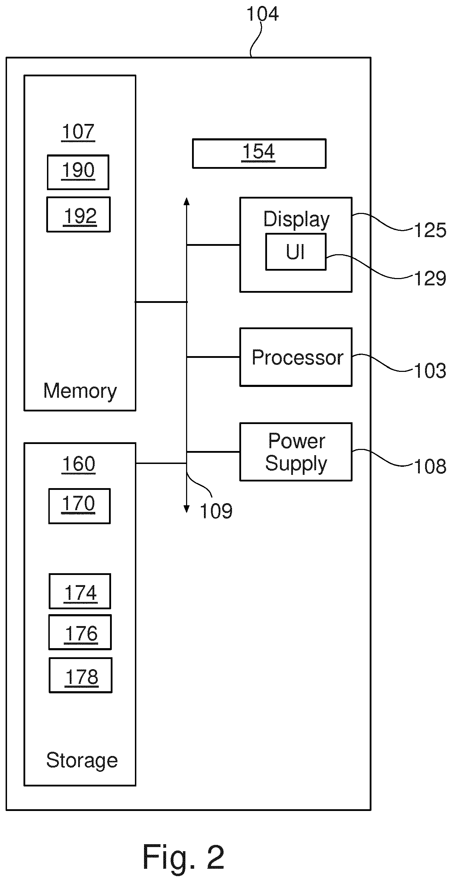

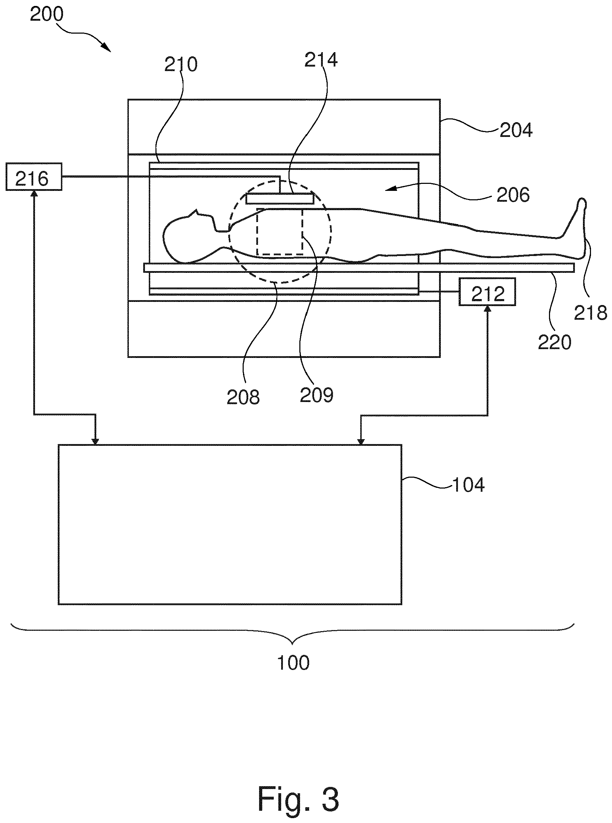

Fig. 2 shows an exemplary computer 104 of a further exemplary embodiment of a magnetic resonance imaging data processing system 100 as illustrated in Fig. 3. In addition to the computer 104, the magnetic resonance imaging data processing system 100 may comprise the first magnetic resonance system 200 from which the computer 104 receives the first set of magnetic resonance data 170.

The computer 104 may be operatively connected with the first magnetic resonance system 200 via the hardware interface 154. The connection between hardware interface 154 and the first MRI system may for example comprise a BUS Ethernet connection, WAN connection, Internet connection etc. The computer 104 and the first MRI system 200 may or may not be an integral part. In other terms, the computer 104 may or may not be external to the first MRI system 200. The first MRI system 200 may comprise components that are controlled by the processor 103 in order to configure the first MRI system 200. The configuration of the first MRI system 200 may enable the operation of the respective system, i.e. acquiring magnetic resonance imaging data. The operation of the first MRI system 200 may for example be automatic. The first MRI system 200 may be configured to provide output data such as the first set of magnetic resonance imaging data 170 to computer 104 in response to a magnetic resonance imaging data acquisition instruction executed by the processor 103.

The processor 103 may be adapted to receive the first set of magnetic resonance imaging data 170 from the first MRI system 200 in a compatible digital form so that such magnetic resonance imaging data 170 may be processed and images 178 reconstructed using the received MRI data 170 may be displayed on the display device 125. Furthermore, additional information may be received from the first MRI system 200 in a compatible digital form so that it may be displayed on the display device 125. Such additional information may include operating parameters, alarm notifications, and other information related to the use, operation and function of the first MRI system.

Storage device 160 is shown as containing the first set of magnetic resonance imaging data 170 acquired by the first magnetic resonance imaging system 200. The first set of magnetic resonance imaging data 170 is characterized by a first set of imaging characteristics. The first set of imaging characteristics comprises one or more of the following: a first signal-to-noise-ratio, a first image contrast, a first image distortion and a first chemical shift.
The storage device 160 is shown as further containing a set of definitions 174 defining second imaging characteristics characterizing second magnetic resonance imaging data 172 acquirable by a second magnetic resonance imaging system different from the first magnetic resonance system. The second set of imaging characteristics comprises one or more of the following: a second signal-to-noise-ratio, a second image contrast, a second image distortion and a second chemical shift. The storage device 160 is shown as further containing a set of emulated magnetic resonance imaging data 176. The set of emulated magnetic resonance imaging data 176 results from applying a post-processing filtering operation to the first magnetic resonance imaging data 170. The set of emulated magnetic resonance imaging data 176 is characterized by a set of emulated imaging characteristics. The set of emulated imaging characteristics comprises one or more of the following: an emulated signal-to-noise-ratio, an emulated image contrast, an emulated image distortion and an emulated chemical shift. The emulated set of imaging characteristics mimics a second set of imaging characteristics as defined by the set of definitions 174. The storage device 160 is shown as further containing a first magnetic resonance image 178 reconstructed using the set of emulated magnetic resonance imaging data 176. The computer 104 may be configured to provide the emulated set of magnetic resonance imaging data and/or the first magnetic resonance image for a comparison with a second set of magnetic resonance imaging data, the comparison comprising displaying the first magnetic resonance image and a second magnetic resonance image reconstructed using the second magnetic resonance imaging data. For this purpose, the computer system 104 may store the emulated set of magnetic resonance imaging data and/or the first magnetic resonance image on a portable, tangible computer readable-storage medium or transfer the emulated set of magnetic resonance imaging data and/or the first magnetic resonance image via a network to a further computer system and/or display device for displaying.

The memory 107 is shown as containing a post-processing filtering module 190. The post-processing filtering module 190 contains machine executable instructions an execution of which by the processor may cause the processor 103 to execute a post-processing filtering operation on the first set of magnetic resonance imaging data 170. The memory 107 is shown as further containing a magnetic resonance imaging system control module 192. The control module 192 contains machine executable instructions which enables the processor 103 to control the magnetic resonance imaging system 200.

Fig. 3 shows an example of a first magnetic resonance imaging system 200 for acquiring the first set of magnetic resonance imaging data. The first magnetic resonance imaging system 200 comprises a magnet 204. The magnet 204 is a superconducting cylindrical type magnet with a bore 206 through it. The use of different types of magnets is also possible. For instance, it is also possible to use both a split cylindrical magnet and a so called open magnet. A split cylindrical magnet is similar to a standard cylindrical magnet, except that the cryostat has been split into two sections to allow access to the axial plane through the iso-center of the magnet, such magnets may for instance be used in conjunction with charged particle beam therapy. An open magnet has two magnet sections, one above the other with a space in-between that is large enough to receive a subject: the arrangement of the two sections area similar to that of a Helmholtz coil. Open magnets are popular, because the subject is less confined. Inside the cryostat of the cylindrical magnet there is a collection of superconducting coils. Within the bore 206 of the cylindrical magnet 204 there is an imaging zone 208 where the magnetic field is strong and uniform enough to perform magnetic resonance imaging. A region of interest 209 is shown within the imaging zone 208. The magnetic resonance data is typically acquired for the region of interest. A subject 218 is shown as being supported by a subject support 220 such that at least a portion of the subject 218 is within the imaging zone 208 and the region of interest 209.

Within the bore 206 of the magnet there is also a set of magnetic field gradient coils 210 which is used for acquisition of magnetic resonance data to spatially encode magnetic spins within the imaging zone 208 of the magnet 204. The magnetic field gradient coils 210 are connected to a magnetic field gradient coil power supply 212. The magnetic field gradient coils 210 are intended to be representative. Typically, magnetic field gradient coils 210 contain three separate sets of coils for spatially encoding in three orthogonal spatial directions. A magnetic field gradient power supply supplies current to the magnetic field gradient coils. The current supplied to the magnetic field gradient coils 210 is controlled as a function of time and may be ramped or pulsed.

Adjacent to the imaging zone 208 is a radio-frequency coil 214 for manipulating the orientations of magnetic spins within the imaging zone 208 and for receiving radio transmissions from spins also within the imaging zone 208. The radio frequency antenna may contain multiple coil elements. The radio frequency antenna may also be referred to as a channel or antenna. The radio-frequency coil 214 is connected to a radio frequency transceiver 216. The radio-frequency coil 214 and radio frequency transceiver 216 may be replaced by separate transmit and receive coils and a separate transmitter and receiver. It is understood that the radio-frequency coil 214 and the radio frequency transceiver 216 are representative. The radio-frequency coil 214 is intended to also represent a dedicated transmit antenna and a dedicated receive antenna. Likewise, the transceiver 216 may also represent a separate transmitter and separate receivers. The radio-frequency coil 214 may also have multiple receive/transmit elements and the radio frequency transceiver 216 may have multiple receive/transmit channels. For example, if a parallel imaging technique such as SENSE is performed, the radio-frequency could 214 will have multiple coil elements.

The transceiver 216 and the gradient controller 212 are shown as being connected to the computer system 104 of Fig.2.

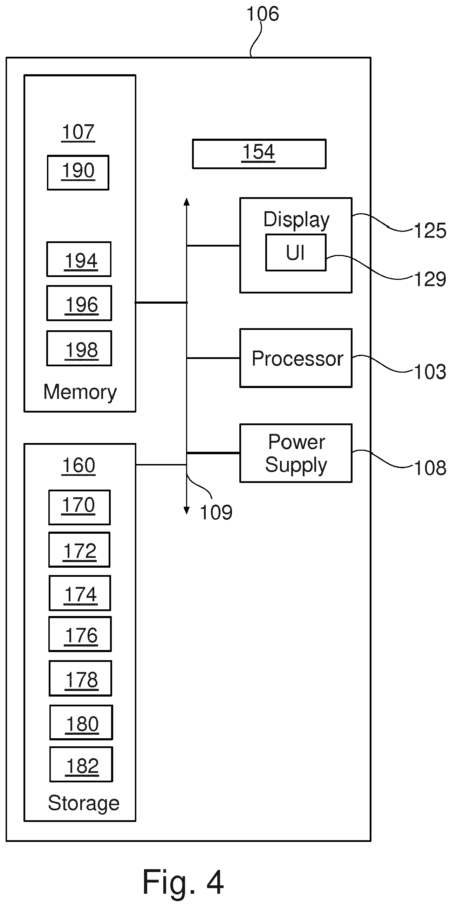

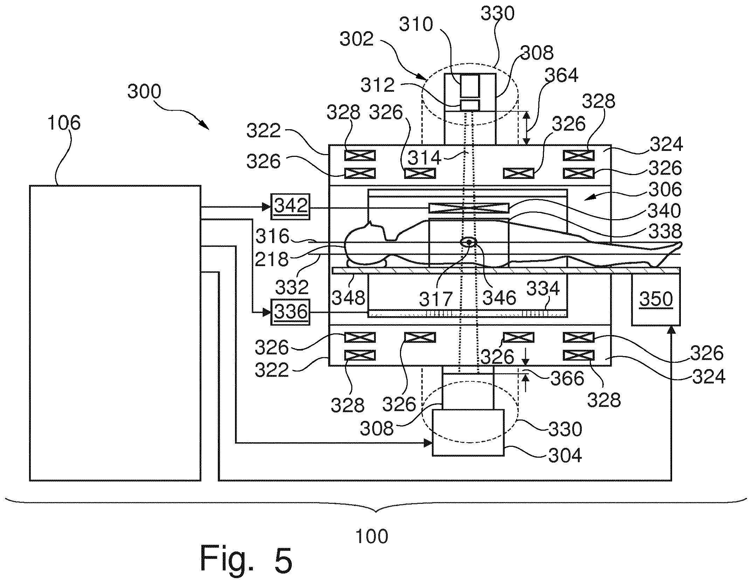

Fig. 4 shows an exemplary computer 106 of a further exemplary embodiment of a magnetic resonance imaging data processing system 100 as illustrated in Fig. 5. In addition to the computer 106, the magnetic resonance imaging data processing system 100 may comprise the second magnetic resonance system 200 in form of a magnetic resonance imaging module 306 of an MRI-guided radiation delivery system 300, from which the computer 104 receives the second set of magnetic resonance data 172.

The computer 106 maybe operatively connected with the MRI-guided radiation delivery system 300 via the hardware interface 154. The connection between hardware interface 154 and the MRI-guided radiation delivery system 300 may for example comprise a BUS Ethernet connection, WAN connection, Internet connection etc. The computer 104 and the MRI-guided radiation delivery system 300 may or may not be an integral part. In other terms, the computer 104 may or may not be external to the MRI-guided radiation delivery system 300. The MRI-guided radiation delivery system 300 may comprise components that are controlled by the processor 103 in order to configure the MRI-guided radiation delivery system 300. The configuration of the MRI-guided radiation delivery system 300 may enable the operation of the respective system, i.e. acquiring magnetic resonance imaging data and deliver radiation. The operation of the MRI-guided radiation delivery system 300 may for example be automatic. The MRI-guided radiation delivery system 300 may be configured to provide output data such as the second set of magnetic resonance imaging data 172 to computer 104 in response to a magnetic resonance imaging data acquisition instruction executed by the processor 103.

The processor 103 may be adapted to receive the second set of magnetic resonance imaging data 172 from the MRI-guided radiation delivery system 300 in a compatible digital form so that such magnetic resonance imaging data 172 maybe processed and images 180 reconstructed using the received MRI data 172 may be displayed on the display device 125. Furthermore, additional information may be received from the MRI-guided radiation delivery system 300 in a compatible digital form so that it may be displayed on the display device 125. Such additional information may include operating parameters, alarm notifications, and other information related to the use, operation and function of the MRI-guided radiation delivery system 300.

Storage device 160 is shown as containing a first set of magnetic resonance imaging data 170 that have been acquired by a first magnetic resonance imaging system, like e.g. magnetic resonance imaging system 200 of Fig. 3. The first set of magnetic resonance imaging data 170 is characterized by a first set of imaging characteristics. The first set of imaging characteristics comprises one or more of the following: a first signal-to-noise-ratio, a first image contrast, a first image distortion and a first chemical shift. The storage device 160 is shown as further containing a second set of magnetic resonance imaging data 172 acquired by the magnetic resonance imaging module 306 of the MRI-guided radiation delivery system 300. The second set of magnetic resonance imaging data 172 is characterized by a second set of imaging characteristics. The second set of imaging characteristics comprises one or more of the following: a second signal-to-noise-ratio, a second image contrast, a second image distortion and a second chemical shift. The storage device 160 is shown as further containing a set of definitions 174 defining the second imaging characteristics characterizing the second set of magnetic resonance imaging data 176. The set of definitions 174 may result from an analysis of the second set of magnetic resonance imaging data 172 by the computer 106 or the set of definitions 174 or may e.g. result from a calibrating processor the MRI-guided radiation delivery system 300. The storage device 160 is shown as further containing a set of emulated magnetic resonance imaging data 176. The set of emulated magnetic resonance imaging data 176 results from applying a post-processing filtering operation to the first magnetic resonance imaging data 170. The set of emulated magnetic resonance imaging data 176 is characterized by a set of emulated imaging characteristics. The set of emulated imaging characteristics comprises one or more of the following: an emulated signal-to-noise-ratio, an emulated image contrast, an emulated image distortion and an emulated chemical shift. The emulated set of imaging characteristics mimicking a second set of imaging characteristics as defined by the set of definitions 174. The storage device 160 is shown as further containing a first magnetic resonance image 178 reconstructed using the set of emulated magnetic resonance imaging data 176 as well as a second magnetic resonance image 180 reconstructed using the second set of magnetic resonance imaging data 172. The storage device is shown as further containing a dose plan 182 comprising e.g. coordinates of a target volume and radiation doses to be applied to the target volume.

The memory 107 is shown as containing a post-processing filtering module 190. The post-processing filtering module 190 contains machine executable instructions an execution of which by the processor may cause the processor 103 to execute a post-processing filtering operation, e.g. on the first set of magnetic resonance imaging data 170. The memory 107 is shown as further containing a radiation delivery system control module 194. The radiation delivery system control module 194 contains machine executable instructions which enable the processor 103 to control the overall functioning of the MRI-guided radiation delivery system 300. The memory 107 is shown as further containing a control module 196 for a radiation delivery module 302 of the MRI-guided radiation delivery system 300. The control module 196 contains machine executable instructions which enables the processor 103 to control the functioning of the radiation delivery module 302. The memory 107 is shown as further containing a control module 198 for controlling an MRI module 306, i.e. MRI system, of the MRI-guided radiation delivery system 300. The control module 198 contains machine executable instructions which enable the processor 103 to control the functioning of the MRI module 306.

Fig. 5 shows a further exemplary embodiment of a magnetic resonance imaging data processing system 100 comprising an MRI-guided radiation delivery system 300. The MRI-guided radiation delivery system 300 is shown as comprising a radiation delivery module 302 and a magnetic resonance imaging module 306. The radiation delivery module 302 comprises a ring mechanism 308. The ring mechanism 308 supports a radiation source 310. The radiation source 310 is representative and may e.g. be a LINAC x-ray source or and a radioisotope gamma radiation source. Adjacent to the radiation source 310 is a multi-leaf beam collimator 312 for collimating a radiation beam 314 that is generated by the radiation source 310. The ring mechanism 308 is also adapted for moving e.g. rotating the radiation source 310 and the beam collimator 312 about a rotational point 317 of the radiation delivery module 302. A rotational axis 316 passes through the rotational point 317.

The magnetic resonance imaging module 306 is shown as comprising a main magnet 322. The ring mechanism 308 is ring-shaped and surrounds the main magnet 322. The main magnet 322 shown in Fig. 5 is a cylindrical type superconducting magnet. However, other magnets are also applicable for embodiments of the invention. The main magnet 322 has a supercooled cryostat 324. Inside the cryostat 324 there is a collection of superconducting coils 326. There are also compensation coils 328 whose current opposes the direction of current in the superconducting coils 326. This creates a low magnetic field zone 330 that circles or encompasses the main magnet 322. The cylindrical main magnet 322 is shown as having an axis 332 of symmetry.

Within the bore of the magnet there is a magnetic field gradient coil 334 which is used for acquisition of image magnetic resonance data to spatially encode objects within an imaging zone 338 of the main magnet 322. The magnetic field gradient coil 334 is connected to a magnetic field gradient coil power supply 336. The magnetic field gradient coil 334 is intended to be representative. Typically, magnetic field gradient coils contain three separate sets of coils for spatially encoding in three orthogonal spatial directions. The imaging zone 338 is located in the center of the main magnet 322.

Adjacent to the imaging zone 338 is a radio frequency (RF) coil 340 for manipulating the orientations of magnetic spins within the imaging zone 338 and for receiving radio transmissions from spins also within the imaging zone 338. The radio frequency coil 340 is connected to a radio frequency transceiver 342. The radio frequency coil 340 and radio frequency transceiver 342 may be replaced by separate transmit and receive coils and a separate transmitter and receiver. It is understood that the radio frequency coil 340 and the radio frequency transceiver 342 are simply representative.

Within the center of the main magnet 322 is also located a subject 218. The subject 218 has a target volume (or target zone) 346 and is shown as reposing on a support 348. The RF coil 340 may transmit RF pulses into the target volume 346. The support 348 has a mechanical positioning system 350. The mechanical positioning system 350 is adapted for positioning the support 348 within the main magnet 322. Depending upon the space available inside of the main magnet 322, the mechanical positioning system 350 may move the support 348 in different directions including a direction perpendicular to the magnet axis 332. If there is more space available inside the main magnet 322 the mechanical positioning system 350 may have more degrees of freedom. For instance, the mechanical positioning system 350 may position the support 348 with six degrees of freedom.

The radio frequency transceiver 342, the magnetic field gradient coil power supply 336, the mechanical actuator 304, and the mechanical positioning system 350 are all shown as being connected to the computer 106 of Fig. 4.
a hardware interface 354 of a computer 311 configured for controlling the radiation delivery system 300.

The radiation delivery module 302 maybe, for example, connected to the hardware interface 154 of computer 106 and communicates with the computer 106 via the mechanical actuator 304 or via other means enabling communication between radiation delivery module 302 and the computer 106.

For the example shown in Fig. 4, the rotational axis 316 of the radiation delivery module is not coaxial with the magnet axis 332. The rotational point 317 is shown as being off center from the magnet axis 332. It can be seen that the target zone 346 is off-center and away from the magnet axis 332. The radiation delivery module 302 has been moved by mechanical actuator 304 such that the rotational point 317 of the radiation delivery module is within the target zone 346. It can be seen that the ring mechanism 308 has been moved relative to the magnet 322.

The radiation beam 314 passes through the rotational point 317. Placing the rotational point 317 at the center of the target zone 346 allows the target zone to be treated continuously when the radiation beam 314 is created by the radiation source 310 and is rotated by the ring mechanism 308.

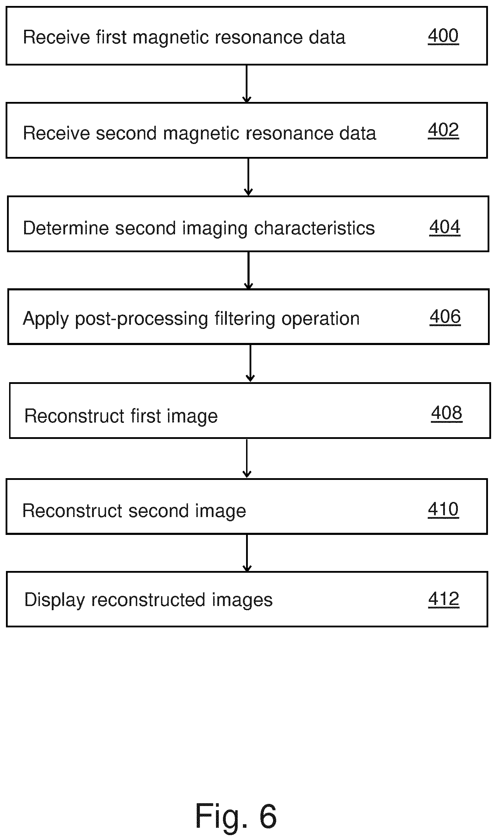

Fig. 6 shows a flowchart which illustrates an exemplary method of controlling the magnetic resonance imaging data processing system 100 of Fig. 1. In step 400, the magnetic resonance imaging data processing system 100 receives first magnetic resonance imaging data acquired by a first magnetic resonance imaging system and characterized by a first set of imaging characteristics. The first set of imaging characteristics comprising one or more of the following: a first signal-to-noise-ratio, a first image contrast, a first image distortion and a first chemical shift. The first magnetic resonance imaging data may e.g. be received from the first magnetic resonance imaging system, from a database, or from a computer readable storage medium. In step 402, the magnetic resonance imaging data processing system 100 receives second magnetic resonance imaging data acquired by a second magnetic resonance imaging system different from the first magnetic resonance system. The second set of imaging characteristics comprising one or more of the following: a second signal-to-noise-ratio, a second image contrast, a second image distortion and a second chemical shift. The second magnetic resonance imaging data may e.g. be received from the second magnetic resonance imaging system, from a database, or from a computer readable storage medium. In step 404, the magnetic resonance imaging data processing system 100 determines the second set of imaging characteristics. The second set of imaging characteristics may e.g. be determined from a set of definitions defining the second set of imaging characteristics. The respective set of definitions may e.g. be received from the second magnetic resonance imaging system, from a database, or from a computer readable storage medium. Alternatively, the respective set of definitions may be generated by the magnetic resonance imaging data processing system 100 analyzing the second magnetic resonance imaging data. In step 406, a post-processing filtering operation is applied to the first magnetic resonance imaging data generating emulated magnetic resonance imaging data. The emulated magnetic resonance imaging data is characterized by a set of emulated imaging characteristics. The set of emulated imaging characteristics comprises one or more of the following: an emulated signal-to-noise-ratio, an emulated image contrast, an emulated image distortion and an emulated chemical shift. The resulting emulated set of imaging characteristics mimics the second set of imaging characteristics defined by the set of definitions. In step 408, a first magnetic resonance image is reconstructed using the emulated magnetic resonance imaging data. In step 410, a second magnetic resonance image is reconstructed using the second magnetic resonance imaging data. In step 412, the reconstructed magnetic resonance images are displayed on a display, thus enabling a comparison of the same.

According to the embodiments, the first and the second magnetic resonance data are each received in form of magnetic resonance images already reconstructed from the magnetic resonance imaging data, e.g. by the first and second MRI systems, respectively. Thus, the post-processing filtering operation may be applied to the received magnetic resonance images representing the first magnetic resonance data resulting in an emulated magnetic resonance image representing emulated magnetic resonance data. According to this embodiment, steps 408 and 410 maybe omitted.

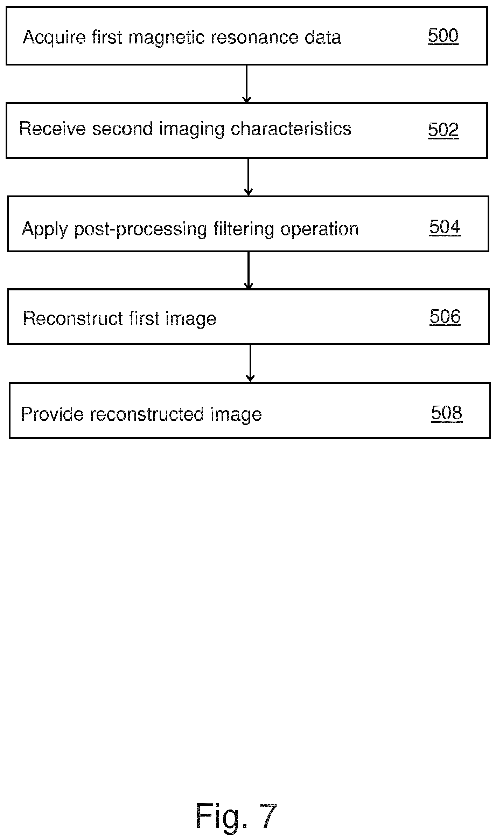

Fig. 7 shows a flowchart which illustrates an exemplary method of controlling the magnetic resonance imaging data processing system 100 of Fig. 3. In step 500, the magnetic resonance imaging data processing system 100 acquires first magnetic resonance imaging data using the first magnetic resonance imaging system comprised by the magnetic resonance imaging data processing system 100. The first magnetic resonance imaging data characterized by a first set of imaging characteristics. The first set of imaging characteristics comprising one or more of the following: a first signal-to-noise-ratio, a first image contrast, a first image distortion and a first chemical shift. In step 502, a second set of magnetic resonance imaging data is received characterizing second magnetic resonance imaging data acquired by a second magnetic resonance imaging system different from the first magnetic resonance system. The second set of imaging characteristics comprising one or more of the following: a second signal-to-noise-ratio, a second image contrast, a second image distortion and a second chemical shift. The second set of imaging characteristics may e.g. be received in form of a set of definitions defining the second set of imaging characteristics. The respective set of definitions may e.g. be received from the second magnetic resonance imaging system, from a database, or from a computer readable storage medium. In step 504, a post-processing filtering operation is applied to the first magnetic resonance imaging data generating emulated magnetic resonance imaging data. The emulated magnetic resonance imaging data is characterized by a set of emulated imaging characteristics. The set of emulated imaging characteristics comprises one or more of the following: an emulated signal-to-noise-ratio, an emulated image contrast, an emulated image distortion and an emulated chemical shift. The resulting emulated set of imaging characteristics mimics the second set of imaging characteristics defined by the set of definitions. In step 508, a first magnetic resonance image is reconstructed using the emulated magnetic resonance imaging data. In step 508, the first magnetic resonance image is provided for a comparison with a second magnetic resonance image reconstructed using the second magnetic resonance imaging data. For example, the first magnetic resonance image is stored on a computer readable storage medium, in a database or sent via a communication connection, e.g. provided by a network, to a display device. For example, the display device is provided by the second MRI system, which e.g. maybe provided by an MRI-guided radiation delivery system. The first magnetic resonance image may e.g. be used for performing a radiotherapy simulation and/or preparing a dose plan. The MRI-guided radiation delivery system may further use the first magnetic resonance image to adjust setting of the MRI-guided radiation delivery system, like e.g. the position of the subject, in accordance with the radiotherapy simulation and/or the dose plan.

According to embodiments, step 506 may be executed before step 504, i.e. the first magnetic resonance image may be reconstructed using the first magnetic resonance data and the post-processing filtering operation may be applied to the first magnetic resonance image. The post-processing filtering operation may thus result in an emulated magnetic resonance image which may be provided in step 508.

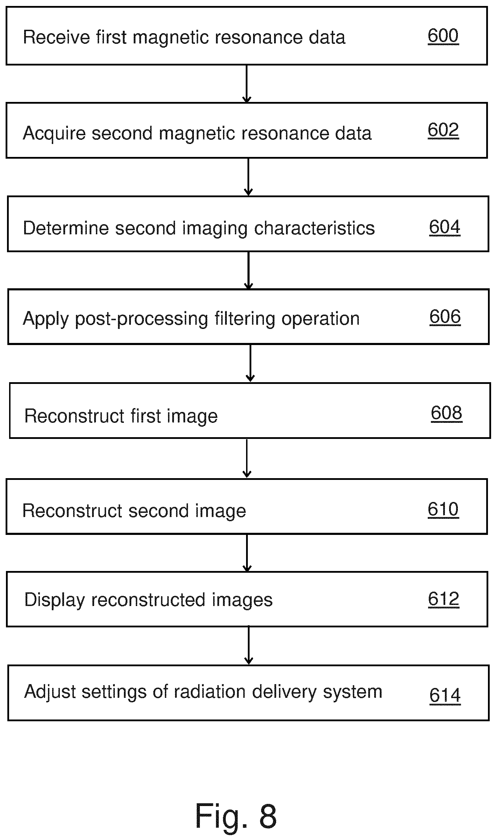

Fig. 8 shows a flowchart which illustrates an exemplary method of controlling the magnetic resonance imaging data processing system 100 of Fig. 5. In step 600, the magnetic resonance imaging data processing system 100 receives first magnetic resonance imaging data acquired by a first magnetic resonance imaging system and characterized by a first set of imaging characteristics. The first set of imaging characteristics comprising one or more of the following: a first signal-to-noise-ratio, a first image contrast, a first image distortion and a first chemical shift. The first magnetic resonance imaging data may e.g. be received from the first magnetic resonance imaging system, from a database, or from a computer readable storage medium. In step 602, the magnetic resonance imaging data processing system 100 acquires second magnetic resonance imaging data using the second magnetic resonance imaging system comprised by the magnetic resonance imaging data processing system 100. The second magnetic resonance imaging system may e.g. be provided by an MRI module of an MRI-guided radiation delivery system. The second magnetic resonance imaging data characterized by a second set of imaging characteristics. The second set of imaging characteristics comprising one or more of the following: a second signal-to-noise-ratio, a second image contrast, a second image distortion and a second chemical shift. In step 604, the magnetic resonance imaging data processing system 100 determines the second set of imaging characteristics. The second set of imaging characteristics may e.g. be determined from a set of definitions defining the second set of imaging characteristics. The respective set of definitions may e.g. be received from the second magnetic resonance imaging system, from a database, or from a computer readable storage medium. Alternatively, the respective set of definitions may be generated by the magnetic resonance imaging data processing system 100 analyzing the second magnetic resonance imaging data. In step 606, a post-processing filtering operation is applied to the first magnetic resonance imaging data generating emulated magnetic resonance imaging data. The emulated magnetic resonance imaging data is characterized by a set of emulated imaging characteristics. The set of emulated imaging characteristics comprises one or more of the following: an emulated signal-to-noise-ratio, an emulated image contrast, an emulated image distortion and an emulated chemical shift. The resulting emulated set of imaging characteristics mimics the second set of imaging characteristics defined by the set of definitions. In step 608, a first magnetic resonance image is reconstructed using the emulated magnetic resonance imaging data. In step 610, a second magnetic resonance image is reconstructed using the second magnetic resonance imaging data. In step 612, the reconstructed magnetic resonance images are displayed on a display, thus enabling a comparison of the same. In step 614, based on the comparison of the first and the second image setting of the MRI-guided radiation delivery system, like e.g. the position of the subject, may be adjusted, e.g. in accordance with a radiotherapy simulation and/or a dose plan based on the first magnetic resonance data.

According to embodiments, the first magnetic resonance data may be received in form of a magnetic resonance image, to which the post-processing filtering operation is applied. Thus, step 606 may directly result in an emulated magnetic resonance image without requiring a further reconstruction like step 608. Alternatively, the reconstruction according to step 08 may be executed before step 606 on the first magnetic resonance data received in step 600. Again, which the post-processing filtering operation may be applied on a magnetic resonance image representing the first magnetic resonance data and thus resulting in an emulated magnetic resonance image without requiring any further reconstructions.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measured cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

### LIST OF REFERENCE NUMERALS

- 100: magnetic resonance imaging system
- 102: computer
- 104: computer
- 103: processor
- 106: computer
- 107: memory
- 108: power supply
- 109: bus
- 125: display
- 129: user interface
- 160: storage
- 170: first set of magnetic resonance imaging data
- 172: second set of magnetic resonance imaging data
- 174: set of definitions
- 176: emulated set of magnetic resonance imaging data
- 178: first magnetic resonance image
- 180: second magnetic resonance image
- 182: dose plan
- 190: post-processing filtering module
- 192: control module
- 194: control module
- 196: control module
- 198: control module
- 162: bottom face
- 200: magnetic resonance imaging system
- 204: magnet
- 206: bore of magnet
- 208: imaging zone
- 209: region of interest
- 210: magnetic field gradient coils
- 212: magnetic field gradient coil power supply
- 214: radio-frequency coil
- 216: transceiver
- 218: subject
- 220: subject support
- 300: MRI-guided radiation delivery system
- 302: radiation delivery module
- 304: mechanical actuator
- 306: magnetic resonance imaging module
- 308: ring mechanism
- 310: radiation source
- 311: computer
- 312: multi-leaf beam collimator
- 314: radiation beam
- 316: rotational axis
- 317: rotational point
- 322: main magnet
- 324: cryostat
- 326: superconducting coil
- 328: compensation coil
- 330: low magnetic field zone
- 332: magnet axis
- 334: magnetic field gradient coil
- 336: magnetic field gradient coil power supply
- 338: imaging zone
- 340: radio frequency coil
- 342: radio frequency transceiver
- 346: target volume
- 348: subject support
- 350: mechanical positioning system

## Claims

1. A magnetic resonance imaging data processing system (100), the magnetic resonance imaging data processing system (100) comprising:
- a memory (107) storing machine executable instructions comprising instructions (190-198) for a post-processing filtering operation,
- a processor (103) for controlling the magnetic resonance imaging data processing system (100), execution of the machine executable instructions (190-198) by the processor (103) causing the processor (103) to control the magnetic resonance imaging data processing system (100) to:
- receive first magnetic resonance imaging data (170) acquired by a first magnetic resonance imaging system (200) and having by a first set of imaging characteristics, the first set of imaging characteristics comprising one or more of the following: a first signal-to-noise-ratio, a first image contrast, a first image distortion and a first chemical shift,
- apply the post-processing filtering operation to the first magnetic resonance imaging data (170) generating emulated magnetic resonance imaging data (176) having a set of emulated imaging characteristics, the set of emulated imaging characteristics comprising one or more of the following: an emulated signal-to-noise-ratio, an emulated image contrast, an emulated image distortion and an emulated chemical shift, the emulated set of imaging characteristics mimicking a second set of imaging characteristics (174) of second magnetic resonance imaging data (172) acquired by a second magnetic resonance imaging system (306) different from the first magnetic resonance system (200), the second set of imaging characteristics (174) comprising one or more of the following: a second signal-to-noise-ratio, a second image contrast, a second image distortion and a second chemical shift,
- provide the emulated magnetic resonance imaging data (176) for a comparison with the second magnetic resonance imaging data (172), the comparison comprising displaying a first magnetic resonance image (178) reconstructed using the emulated magnetic resonance imaging data (176) and a second magnetic resonance image (180) reconstructed using the second magnetic resonance imaging data (172).

2. The magnetic resonance imaging data processing system (100) of claim 1, the magnetic resonance imaging data processing system (100) further comprising a display (125), the execution of the machine executable instructions (190-198) further causing the processor (103) to control the magnetic resonance imaging data processing system (100) to display (125) the first and the second magnetic resonance images (178, 180) on the display.

3. The magnetic resonance imaging data processing system (100) of any of the previous claims, the first magnetic resonance imaging data (170) being received in form of a third magnetic resonance image and the emulated magnetic resonance imaging data (178) being generated in form of the first magnetic resonance image.

4. The magnetic resonance imaging data processing system (100) of any of the previous claims, the generating of the emulated magnetic resonance imaging data (176) with the emulated signal-to-noise-ration comprising adding noise to the first magnetic resonance imaging data (170) in order to reduce the first signal-to-noise-ration and to match the second signal-to-noise-ration.

5. The magnetic resonance imaging data processing system (100) of any of the previous claims, the generating of the emulated magnetic resonance imaging data (176) with the emulated image contrast comprising using a combination of a T1 map and a T2 map acquired by the first magnetic resonance imaging system (200) in order to adjust the first image contrast and to match the second image contrast.

6. The magnetic resonance imaging data processing system (100) of any of the previous claims, the generating of the emulated magnetic resonance imaging data (176) with the emulated chemical shift comprising applying one of the following to adjust the first chemical shift and to match the second chemical shift: a fat suppression and a water suppression.

7. The magnetic resonance imaging data processing system (100) of any of the previous claims, the comparison further comprising performing an image registration of the first and the second magnetic resonance image (178, 180).

8. The magnetic resonance imaging data processing system (100) of any of the previous claims, the first and the second magnetic resonance image (178, 180) depicting an anatomical structure of interest, the first magnetic resonance image (178) defining a spatial reference position of the anatomical structure of interest, the second magnetic resonance image (180) providing a current spatial position of the anatomical structure of interest, the comparison further comprising determining repositioning parameters in order to adjust the current position of the anatomical structure of interest and to match the spatial reference position.

9. The magnetic resonance imaging data processing system (100) of any of the previous claims, the comparison further comprising determining adjustment parameters for adjusting a dose plan for applying a radiation dose to a target comprised by the anatomical structure of interest, the adjustment parameters being configured to compensate for differences between the spatial reference position of the anatomical structure for which the dose plan is defined and the current position of the anatomical structure of interest.

10. The magnetic resonance imaging data processing system (100) of any of the previous claims, the magnetic resonance imaging data processing system (100) further comprising the first magnetic resonance imaging system (200), the first magnetic resonance imaging system (200) comprising:
- a first main magnet (204) for generating a first main magnetic field within a first imaging zone (208) of the first magnetic resonance imaging system (200),
- a first magnetic field gradient system (210) for generating a first spatially dependent gradient magnetic field within the first imaging zone (208),
- a first radio-frequency antenna system (214) configured for acquiring the first magnetic resonance imaging data (170) from the first imaging zone (208),
- the memory (107) further storing first pulse sequence commands (192), wherein the first pulse sequence commands (192) are configured for controlling the first magnetic resonance imaging system to acquire the first magnetic resonance imaging data (170) from the first imaging zone (208),
- the receiving of the first magnetic resonance imaging data (170) comprising acquiring the first magnetic resonance imaging data (170) from the first imaging zone (208) via the radio frequency antenna system (214) using the first pulse sequence commands (192).

11. The magnetic resonance imaging data processing system (100) of any of claim 1 to 10, the magnetic resonance imaging data processing system (100) further comprising the second magnetic resonance imaging system (306), the second magnetic resonance imaging system (306) comprising:
- a second main magnet (322) for generating a second main magnetic field within a second imaging zone (338) of the second magnetic resonance imaging system (306),
- a second magnetic field gradient system (334) for generating a second spatially dependent gradient magnetic field within the second imaging zone (338),
- a second radio-frequency antenna system (340) configured for acquiring the second magnetic resonance imaging data from the second imaging zone (338),
- the memory (107) further storing second pulse sequence commands (198), wherein the second pulse sequence commands (198) are configured for controlling the second magnetic resonance imaging system (306) to acquire the second magnetic resonance imaging data (172) from the second imaging zone,
- execution of the machine executable instructions (198) further causing the processor (103) to control the second magnetic resonance imaging system (306) to:
- acquire the second magnetic resonance imaging data (172) from the second imaging zone (338) via the second radio-frequency antenna system (340) using the second pulse sequence commands (198),
- reconstructing the second magnetic resonance image (180) using the acquired magnetic resonance data (172).

12. The magnetic resonance imaging data processing system (100) of any of the previous claims, the second magnetic resonance imaging system further comprising a radiation source (310) configured for applying one of the following to a target located within the imaging zone: X-rays and gamma rays.

13. The magnetic resonance imaging data processing system (100) of any of the previous claims, a first magnetic field strength of the first main magnetic field generated by the first main magnet (204) of the first magnetic resonance imaging system (200) being larger than a second magnetic field strength of the second main magnetic field generated by the second main magnet (322) of the second magnetic resonance imaging system (306).

14. A method for controlling a magnetic resonance imaging data processing system (100), the magnetic resonance imaging data processing system (100) comprising:
- a memory (107) storing machine executable instructions comprising instructions (190-198) for a post-processing filtering operation,
- a processor (103) for controlling the magnetic resonance imaging data processing system (100), execution of the machine executable instructions (190-198) by the processor (103) causing the processor (103) to control the magnetic resonance imaging data processing system (100) to execute the method comprising:
- receiving first magnetic resonance imaging data (170) acquired by a first magnetic resonance imaging system (200) and having a first set of imaging characteristics, the first set of imaging characteristics comprising one or more of the following: a first signal-to-noise-ratio, a first image contrast, a first image distortion and a first chemical shift,
- applying the post-processing filtering operation to the first magnetic resonance imaging data (170) generating emulated magnetic resonance imaging data (176) having a set of emulated imaging characteristics, the set of emulated imaging characteristics comprising one or more of the following: an emulated signal-to-noise-ratio, an emulated image contrast, an emulated image distortion and an emulated chemical shift, the emulated set of imaging characteristics mimicking a second set of imaging characteristics (174) of second magnetic resonance imaging data (172) acquired by a second magnetic resonance imaging system (306) different from the first magnetic resonance system (200), the second set of imaging characteristics (174) comprising one or more of the following: a second signal-to-noise-ratio, a second image contrast, a second image distortion and a second chemical shift,
- providing the emulated magnetic resonance imaging data (176) for a comparison with the second magnetic resonance imaging data (172), the comparison comprising displaying a first magnetic resonance image (178) reconstructed using the emulated magnetic resonance imaging data (176) and a second magnetic resonance image (180) reconstructed using the second magnetic resonance imaging data (172).

15. A computer program product comprising machine executable instructions for execution by a processor (103) controlling a magnetic resonance imaging data processing system (100), the machine executable instructions comprising instructions (190-198) for a post-processing filtering operation, execution of the of the machine executable instructions (190-198) by the processor (103) causing the processor (103) to control the magnetic resonance imaging data processing system (100) to:
- receive first magnetic resonance imaging data (170) acquired by a first magnetic resonance imaging system (200) and having a first set of imaging characteristics, the first set of imaging characteristics comprising one or more of the following: a first signal-to-noise-ratio, a first image contrast, a first image distortion and a first chemical shift,
- apply the post-processing filtering operation to the first magnetic resonance imaging data (170) generating emulated magnetic resonance imaging data (176) having a set of emulated imaging characteristics, the set of emulated imaging characteristics comprising one or more of the following: an emulated signal-to-noise-ratio, an emulated image contrast, an emulated image distortion and an emulated chemical shift, the emulated set of imaging characteristics mimicking a second set of imaging characteristics (174) of second magnetic resonance imaging data (172) acquired by a second magnetic resonance imaging system (306) different from the first magnetic resonance system (200), the second set of imaging characteristics (174) comprising one or more of the following: a second signal-to-noise-ratio, a second image contrast, a second image distortion and a second chemical shift,
- provide the emulated magnetic resonance imaging data (176) for a comparison with the second magnetic resonance imaging data (172), the comparison comprising displaying a first magnetic resonance image (178) reconstructed using the emulated magnetic resonance imaging data (176) and a second magnetic resonance image (180) reconstructed using the second magnetic resonance imaging data (172).
